# EUROPEAN PATENT APPLICATION

(11) **EP 3 733 299 A1**
(43) Date of publication of application: **04.11.2020**
(21) Application number: 19171822.0
(22) Date of filing: 30.04.2019
(51) Int. Cl.: B05B 17/00, A61M 11/00, A61M 15/00

(54) **MEMBRANE UNIT FOR GENERATING IN AEROSOL IN AN AEROSOL THERAPY DEVICE, AEROSOL THERAPY DEVICE AND METHOD OF MANUFACTURING A MEMBRANE UNIT OF AN AEROSOL GENERATOR**

(71) Applicant: PARI Pharma GmbH, 82319 Starnberg (DE)
(72) Inventor: Anzenberger, Hans-Lukas, 80337 München (DE); Schlun, Martin, 82031 Grünwald (DE); Seifert, René, 82418 Murnau (DE)
(74) Representative: Hoffmann Eitle

(57) **Abstract**

The present invention relates to a membrane unit for generating an aerosol in an aerosol therapy device, the membrane unit comprising a membrane (1) having an area (6) comprising a plurality of through-holes for nebulizing a fluid and a flange (7) circumferentially surrounding the area (6), and a substrate (2) having an opening (8) wherein the flange (7) of the membrane (1) is welded to the substrate (2) so that the area (6) is located in the opening (8) wherein the weld (5) comprises at least three discontinuous welds (5c) arranged at a distance from each other along a circumference of the opening (8) and a first annular weld (5b) circumferentially surrounding the opening (8).

## Description

### Technical Field

The invention relates to a membrane unit for generating an aerosol in an aerosol therapy device, the membrane unit comprising a membrane and a substrate. The invention also relates to an aerosol therapy device for generating an aerosol and comprising the membrane unit. Further, the invention relates to a method of manufacturing a membrane unit of an aerosol generator.

### Prior Art

Membrane nebulizers or membrane units for generating liquid droplets using an oscillating membrane, in particular for nebulising fluid, in particular liquids for therapeutic purposes are known, for example from DE 10 2009 026 363 A1, which is incorporated by reference in its entirety.

During production of such membrane units, the membrane must be connected to an actuator that causes the membrane to oscillate. This can be realised by connecting the membrane to a substrate (also called carrier or support), by adhering or gluing, for example. The actuator causes the membrane to oscillate and, thus, the fluid is nebulised through through-holes in the membrane from one side to the other. It is also known that electric resistance welding can be used for attaching the membrane to the substrate or carrier, and that laser welding is an alternative, according to DE 10 2009 026 636 A1.

It is important that membrane units have appropriate oscillation behaviour and, thus, allow for appropriate aerosol generation, which is significantly influenced by the type and way of connection between the membrane and the substrate. Also heat production when connecting the membrane and the substrate can be an issue, which could lead to distortion or lack of sealing (leakage) between the membrane and the substrate. This would adversely influence the oscillation behaviour and aerosol generation. If the weld is not appropriately formed, a gap between the membrane and the substrate can be formed, which is likely to form a weak point for corrosion. Prior art membrane units fail to provide satisfactory results in this regard.

### Description of the Invention

Hence, the object forming the basis of the invention is to provide a high-quality membrane unit which provides appropriate oscillation behaviour, wherein this is achieved in a simple, quick and cost-effective manner, in particular as regards the manufacturing process.

This object is solved by the invention defined in claim 1. Accordingly, a membrane unit for generating an aerosol in an aerosol therapy device is provided, wherein the membrane has an area comprising a plurality of through-holes for nebulizing a fluid, and a flange circumferentially surrounding the area. The membrane unit further comprises a substrate having an opening, wherein the flange of the membrane is welded to the substrate so that the area is located in the opening, wherein the weld comprises at least three discontinuous welds arranged in a distance to each other along a circumference of the opening and first annular weld circumferentially surrounding the opening.

The object is also solved by the features of claim 6, namely a membrane unit for generating an aerosol in an aerosol therapy device, wherein the membrane unit comprises a membrane having an area comprising a plurality of through-holes for nebulizing a fluid, and a flange circumferentially surrounding the area. Further, the membrane unit comprises a substrate having an opening wherein the flange of the membrane is welded to the substrate so that the area is located in the opening, wherein the weld comprises a first annular weld circumferentially surrounding the opening and a second annular weld, in particular also circumferentially surrounding the opening and, substantially concentric with the first annular weld.

By providing a first annular weld and at least three discontinuous welds and/or a second annular weld, a suitable way for avoiding a gap between the membrane and the substrate can be provided. Hence, corrosion can efficiently be avoided. In particular, the annular weld that circumferentially surrounds the opening ensures that the membrane is sealingly connected to the substrate, along its entire circumference. The additional weld which also extends along the circumference (in the form of three discontinuous welds or an additional annular weld) ensures optimal connection between the membrane and the substrate.

In case at least three discontinuous welds are provided, they can, during manufacture of the membrane units, be formed in a first step, so as to attach the membrane to the substrate, and then, in a second step, the annular weld is formed. This has the advantage that the membrane is, when welding the annular weld, restricted by the at least three discontinuous welds, so that the membrane will not deform during the further welding process and will not create a gap.

Preferably, laser welding is used for providing the welds defined in claims 1 and 6. However, it is also conceivable that the membrane units of claims 1 and 6 are manufactured by using resistance welding, medium frequency welding or capacitor discharge welding, for example. In any of the membrane units according to the invention, the welds can be laser welds.

Preferably, an annular weld circumferentially surrounding the opening means that the membrane is welded around its entire circumference to the substrate. Put differently, the annular weld is a continuous weld that extends around the entire opening. This means that the annular weld is not interrupted along the circumference of the opening.

A discontinuous weld can have the form of a dot. The discontinuous welds can be regarded as discrete welds. In particular, embodiments having at least three, five, eight and ten discontinuous welds are conceivable. For example, a discontinuous or discrete weld may have an area of less than 0.1 mm², preferably about 0.001 to 0.080 mm² or 0.002 to 0.020 mm², more preferably of 0.002826 mm² to 0.070650 mm² and most preferably about 0.002826 mm² to 0.020096 mm². It may be approximately dot-shaped, preferably with a diameter between 0.02 mm and 0.15 mm and more preferably between 0.03 mm and 0.08 mm.

There may be an overlapping of the flange and the substrate. In particular, this means that the flange and the substrate are at least partially located one above the other. For example, a discontinuous or discrete weld may have a dimension/length of about one quarter, or between one third and one fifth of the overlapping. The overlapping may have for example about 0.1 to 1.0 mm and is preferably about 0.2 mm. These dimensions preferably relate to the length in the radial dimension, in relation to the central axis of the membrane unit.

In general, an annular weld may have a radius of about 2 mm to 10 mm. An outer annular weld may have a radius preferably about 3.0 mm to 8.0 mm and most preferably about 4.0 to 6.0 mm. An inner annular weld may have a radius preferably from 2.8 mm to 7.8 mm and most preferably from 3.8 to 5.8 mm. This allows for optimal oscillation and avoidance of heat damage. The radius of an annular weld is preferably measured from the central axis to the middle of the weld measured in the radial direction.

The present invention also solves the object by providing a method of manufacturing a membrane unit of an aerosol generator, wherein the method comprises the following steps, as defined in claim 10: Bringing a substrate having an opening, and a membrane having an area comprising a plurality of through-holes for nebulizing a fluid and a flange circumferentially surrounding the area in surface contact so that the area is located in the opening, and laser welding the flange to the substrate at a first annular weld circumferentially surrounding the opening, wherein optionally a feed rate of the laser along the annular path of the first annular weld is between 200 and 800 mm/s, preferably between 500 to 700 mm/s, most preferably about 600 mm/s, and a laser output is between 300 W and 900 W, preferably between 400 and 800 W, most preferably between 550 to 750 W.

An additional advantage of performing laser welding is that it is contactless, fast and allows for reliable connection between the substrate and the membrane. It allows for a connection which is not susceptible to gaps or corrosion. As explained above, the annular weld also supports a reliable connection between the membrane and the substrate. In particular if the laser output is between 300 W and 900 W and the feed rate is between 200 and 800 mm/s, it is possible to satisfactorily connect the membrane and the substrate and to avoid that the membrane is deformed due to heat absorption. Hence, by means of these parameters for laser welding, an optimal connection between the substrate and the membrane can be obtained, without damaging the membrane.

In particular for the mentioned laser output, the laser wavelength may be between 800 and 1200 nm and preferably between 900 and 1100 nm. The laser may be a solid state laser, for example a laser "InGaAs" with a wavelength between 904 to 1065 nm. In a further example, a YAG (Yttrium-Aluminium-Granat) laser with wavelengths from 946 nm up to 1064 nm may be used.

A further advantage is that, for laser welding, no wearing parts, such as electrodes when using electrical resistance welding, are involved. Due to the high power input by means of a laser, short process times are possible.

A continuous or pulsed laser may be used. Preferably, the pulse width is 0.5 to 2.0 ms, and the pulse frequency is between 100 and 200 Hz.

Preferably, the method according to the invention is used to manufacture the membrane unit of the invention.

The invention is based upon the idea of forming at least one annular weld that circumferentially surrounds the opening and preferably to provide an additional weld, such as at least three discrete welds or an additional annular weld, along the circumference of the opening, wherein the welds may be formed by laser welding, wherein in particular the laser output may be between 300 W and 900 W and the feed rate may be between 200 and 800 mm/s. The result is that a seal for providing an appropriate connection between the membrane and the substrate by means of the annular weld can be achieved, in particular avoiding escape of fluid, in particular of medicament, during nebulization. Further advantages may be a robust and durable mechanical connection of the membrane to the substrate, in particular avoiding corrosion of the membrane and the substrate.

Preferred embodiments are defined in the dependent claims and are described in the following.

Preferably, the membrane unit having a first annular weld and at least three discontinuous welds is formed such that the discontinuous welds are arranged on a common circle, which is concentric with the first annular weld. This provides for a uniform weld contact along the circumference of the opening, in particular when the discontinuous welds are equidistantly arranged.

In a preferred embodiment, the first annular weld is arranged radially inward of the discontinuous welds with respect to the opening. In this embodiment, the first annular weld is inwardly arranged to the discontinuous welds, which ensures that fluid does not enter a space or gap between the membrane flange and the substrate. Hence, a seamless seal and a more reliable connection between the membrane and the substrate can be obtained.

In a further preferred embodiment, the first annular weld is arranged radially inward of the discontinuous welds with respect to the opening. In this embodiment, the first annular weld is closer to the opening than the discontinuous welds, which ensures that fluid does not enter a space or gap between the membrane and the substrate close in parts. Hence, a seamless seal and a more reliable connection between the membrane and the substrate can be obtained.

It is also contemplated to provide a second annular weld substantially concentric with the first annular weld, when a number of discontinuous welds are provided. This increases the bonding strength between the membrane and the substrate and, thus, the reliability of the membrane unit.

The second annular weld can be arranged radially outward of the discontinuous welds. In this case, during manufacture, the heat absorption during welding of the second annular weld is reduced, which avoids damage to the membrane.

In any embodiment having a second annular weld, the second annular weld may overlap a radial outer edge of the flange in a plan view and/or the first annular weld may overlap a radial inner edge of the substrate at the opening in a plan view. Overlap of an annular weld at an edge allows for optimal seamless seal and avoidance of a gap between the membrane and the substrate which may be prone to corrosion when in contact with salt containing liquids. If the first and second annular welds are positioned accordingly, it is possible to eliminate or minimize/reduce the gap on both sides to a minimum, i. e. the substrate opening and the membrane outer flange circumference. This increases the reliability of the membrane unit.

When manufacturing a membrane unit, it is particularly preferable to weld the flange to the substrate at three discrete welds which are arranged around the opening in a circumferential direction, before laser welding the first annular weld. If such single discrete welds are used for positioning and fixing the membrane relative to the substrate, deformation of the membrane due to heat absorption in the membrane when subsequently forming an annular weld can be avoided, as explained above.

It is preferable to bring the membrane into surface contact with the side of the substrate on which nebulization or aerosol generation takes place.

Preferably, a laser which is used for laser welding is a multimode laser having a scanner optic. Compared to a single mode laser and solid state optic, the welding connection can be improved. Also a solid state laser is conceivable.

Optionally, at least one of the surfaces of the substrate and the flange of the membrane to be brought in contact is roughened by a laser structuring. By means of such step before welding the substrate and the membrane, the bonding strength can be increased.

If the substrate is thicker than the flange of the membrane in a direction perpendicular to the surfaces of the substrate and the flange of the membrane to be brought in surface contact, laser welding can be performed from a side of the flange of the membrane. Alternatively, if the substrate is thinner than the flange of the membrane in a direction perpendicular to the surfaces of the substrate and the flange of the membrane to be brought in surface contact (i.e. the vertical direction), laser welding can be performed from a side of the substrate, such as the from the surface area of the substrate. This allows for efficient welding.

The present invention also refers to a method for manufacturing an aerosol therapy device for generating an aerosol, the method comprises the steps of manufacturing a membrane unit of an aerosol generator and additionally the step of positioning the membrane unit in the aerosol therapy device.

In general, the membrane and the substrate may be relatively thin. For example, the thickness of the membrane is between 25 µm and 200 µm, and the thickness of the substrate is between 50 µm and 500 µm. In a preferred example, the thickness of the membrane is between 50 µm and 150 µm, and the thickness of the substrate is between 100 µm and 400 µm. The membrane and/or the substrate may be made of metal and preferably of stainless steel, to ensure an extended persistence.

As to the welding thickness, it is preferable that the weld is not continuous in the vertical direction, i.e. in the direction along which the substrate and the membrane/flange may overlap. It is conceivable that the weld only partially penetrates the membrane and/or substrate. In particular, one of the membrane and the substrate may only partially be penetrated. In a preferred embodiment, the laser may weld the membrane and/or the substrate not completely and preferably less than the half of the total thickness of one or both materials (i.e. of the metal sheets). Put differently, the extension of the weld in the vertical direction is preferably less than half of the total extension of the membrane and/or of the substrate in the vertical direction in the overlapping.

In a further preferred embodiment, the laser should weld the membrane for about one-tenth of its material thickness and/or the substrate for about three-tenths of its material thickness. In another preferred embodiment, the laser should weld the membrane for about three-tenths of its material thickness and/or the substrate for about one-tenth of its material thickness. In an even further preferred embodiment, the laser should weld the membrane for the entirety of its material thickness and the substrate for about three-tenths of its material thickness. In an even further preferred embodiment, the laser should weld the membrane for about three-tenths of its material thickness and the substrate for entirely of its material thickness.

Additionally or alternatively, the laser should weld the membrane for about the entirety of its material thickness and/or the substrate for about 5 µm to 15 µm of its material thickness. In a further preferred embodiment, the laser should weld the membrane for about 5 µm to 15 µm of its material thickness and/or the substrate for the entirety of its material thickness.

An actuator may be provided to cause at least the membrane for nebulising the fluid to oscillate, whereby the actuator may form the substrate or may be connected, for example adhered, to the substrate. It may be arranged on the same side as the membrane or on the opposite second side of the substrate.

Furthermore, the actuator is preferably a piezo-ceramic actuator, in particular a piezo oscillator. The wall thickness of the actuator is thereby of a comparable size and is preferably less than 500 µm, preferred between 25 µm and 500 µm and most preferred between 100 µm and 400 µm.

Actuators other than piezoelectric actuators may likewise also be used, such as, for example, shape memory alloys, oscillating pistons, pump motors, pump pistons, piezo motors, electromagnets with an oscillating core, relays or the like.

The term "substantially" indicates that not only embodiments in which a requirement is exactly fulfilled, but also embodiments where a requirement is approximately or basically fulfilled are encompassed.

### Brief Description of the Drawings

- Fig. 1: shows a cross-section of a membrane unit;
- Fig. 2a: shows a plan view of a membrane unit according to the invention;
- Fig. 2b: shows a plan view of an alternative membrane unit according to the invention;
- Fig. 2c: shows a plan view of a further alternative membrane unit according to the invention;
- Fig. 3a: shows an enlarged cross-section of a membrane unit according to the invention; and
- Fig. 3b: shows an enlarged cross-section of an alternative membrane unit according to the invention.

### Detailed description of the preferred embodiments of the invention

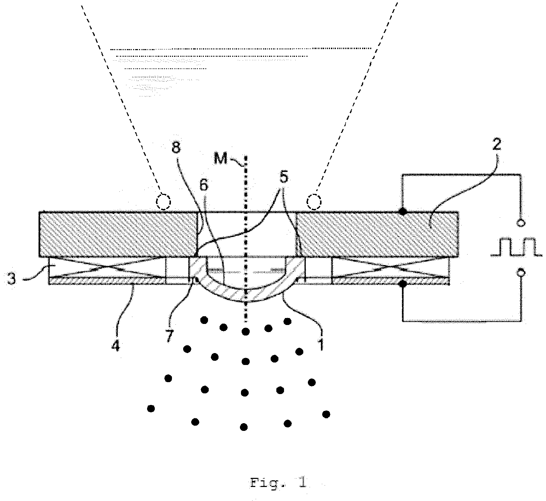

Fig. 1 shows a membrane unit of the present invention. The membrane unit is basically an oscillatable system, which is rotationally symmetrical relative to the central axis M shown in Fig. 1. The membrane unit comprises a membrane 1 and a substrate or carrier or support 2 having a centrally arranged circular opening 8. The membrane 1 is preferably curved or dome-shaped. The membrane 1 is circular and arranged concentric to the central axis M. The membrane 1 comprises a circular, centrally arranged effective area 6 which comprises a plurality of (invisible) through-holes. Preferably, the through-holes are in the range of less than 10 µm, preferably less than 5 µm and more preferably between 1.5 µm and 5 µm in diameter.

A flange or annular collar 7 is arranged concentric to the effective area 6. The flange 7 circumferentially surrounds the area 6. The flange 7 protrudes over the opening 8 (so as to form an overlapping with the substrate 2) and serves to fix the membrane 1 to the substrate 2. The flange of the membrane 1 is welded to the substrate 2 so that the area 6 is located in the opening 8, by means of one or more welds 5.

Fig. 1 shows the membrane unit according to the invention, including a piezo electric element as actuator. When installed in an aerosol therapy device, the fluid to be nebulized is present on the upper side of the substrate 2, and nebulization or aerosol generation occurs on the opposite side, namely the lower side of the substrate 2, when the membrane is caused to oscillate, and the fluid, in particular a liquid, is nebulized through the plurality of through-holes on the lower side of the substrate 2.

A piezo element 3 is attached, in particular adhered, to the substrate 2 on the lower side. An AC voltage can be applied via a first electrode 4 and via the substrate 2. The substrate 2 can assume the function of a second electrode for the piezo element 3. Alternatively, a second electrode may be provided on the upper side of the substrate 2. In one embodiment, the first electrode 4 may be formed as a Kapton foil with electronic conductive paths.

When the AC voltage is applied to the electrodes (see the right part of Fig. 1), this leads to a lengthening and shortening of the piezo element 3 in a direction perpendicular to the axis of symmetry M as shown in Fig. 1. As a result, during the alternating lengthening and shortening of the piezo element 3, the carrier is bent and caused to flexurally oscillate, these oscillations being transferred to the membrane 1. The resonance frequencies of the oscillation system are determined by the membrane 1, the substrate 2 and the piezo element 3 as well as by the type of fixing or connecting between the membrane 1 and the substrate 2. The resonance frequencies of the oscillation system are also influenced by the liquid which is in contact with the membrane 1.

The weld or welding seal 5 in the region of the flange 7 fixes the membrane 1 to the substrate 2. For this purpose, the flange 7 is in surface contact with the lower side of the substrate 2. Connection is carried out such that the membrane 1 with the collar or flange 7 is brought into surface contact with the substrate 2, more specifically the lower side, i.e. lower surface area, of the substrate, and then a welding process is performed.

The width of the flange is adjusted accordingly in the radial direction. For example, the area of the flange is in the range between 5 mm² and a maximum of 96 mm², preferably a maximum of 80 mm², and mostly preferred a maximum of 20 mm². The area of the flange 7 is measured in the region which protrudes over the opening 8 of the substrate 2.

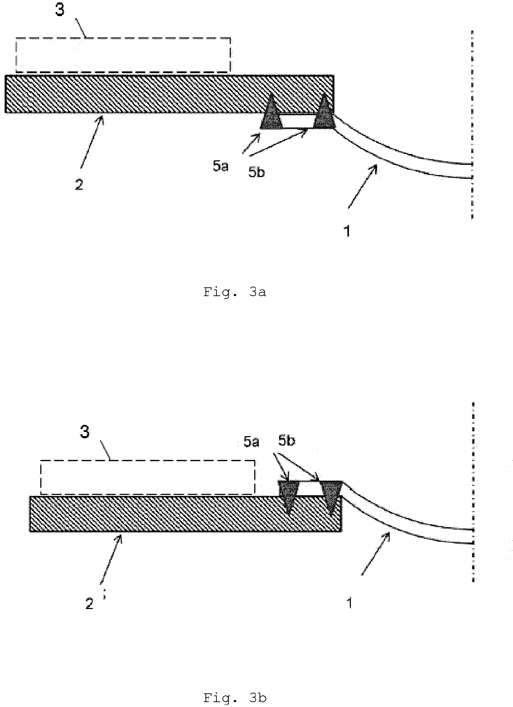

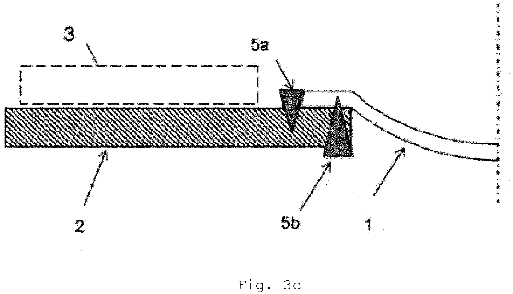

In the embodiment of Fig. 1, the membrane 1 is connected to the lower side of the substrate 2. Also the piezo electric element 3 is connected to the lower side of the substrate 2. The membrane 1 and the piezo electric element 3 are connected to the identical(same) side of the substrate 2. The membrane 1 with the collar or flange 7 is brought into surface contact with the substrate 2, more specifically to the same side as the piezoelectric element is in contact with the substrate 2 (Fig. 1, Fig. 3b, Fig. 3c).

In an alternatively embodiment of Fig. 3a, the membrane 1 is connected to the lower side of the substrate 2 and the piezoelectric element 3 may be connected to the upper side of the substrate 2. The membrane 1 is connected to the opposite side of the substrate 2 than the piezoelectric element 3. The membrane 1 with the collar or flange 7 is brought into surface contact with the substrate 2, more specifically to the opposite side as the piezoelectric element is in contact with the substrate 2.

A laser beam may be applied to the membrane and the substrate as indicated in Fig. 3a, Fig. 3b and Fig. 3c with the triangles marked with 5a and 5b. For example as shown in Fig. 3c, the laser beam may be applied at the triangle 5b such that the laser beam is entirely on the substrate 2. Alternatively, the laser beam could be placed half on the substrate 2 and half on the membrane 1, as indicated by the laser beam at the triangle 5a pointing to the edge of the flange 7. Ideally, one third of the width of the laser beam is on the substrate 2, and two thirds are on the membrane 1.

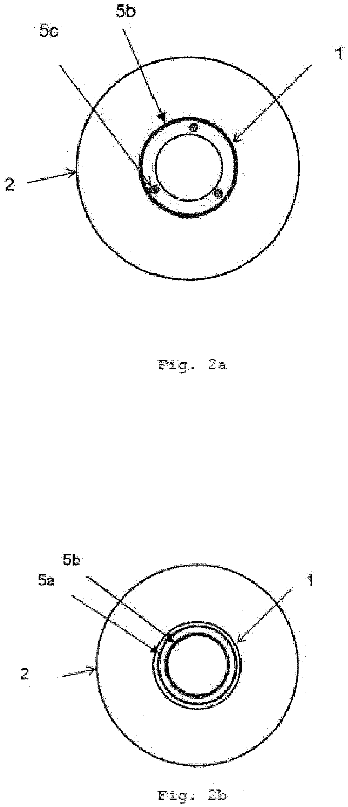

Fig. 2a shows a first specific embodiment in line with the invention, in which the weld 5 comprises three discontinuous or discrete welds 5c arranged equidistantly to each other along a circumference of the opening 8 and a first annular weld 5b circumferentially surrounding the opening. As can be taken from Fig. 2a, the discontinuous welds 5c are arranged on a common circle concentric with the first annular weld 5b. The discontinuous welds 5c fix the substrate 2 to the membrane 1 and may reduce the bulge.

Fig. 2b shows another embodiment of the present invention, in which the weld 5 comprises the first annular weld 5b circumferentially surrounding the opening and a second annular weld 5a substantially concentric with the first annular weld 5b. In particular, also the second annular weld 5a circumferentially surrounds the opening 8.

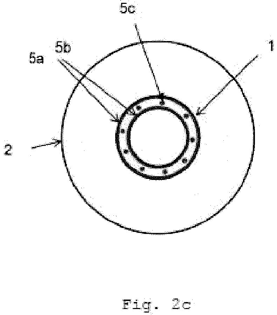

Fig. 2c basically shows a combination of the embodiments of Figures 2a and 2b, which can, however, also be provided alternatively. More specifically, in Fig. 2c, nine discontinuous welds 5c, a first annular weld 5b, and a second annular weld 5a are provided. The second annular weld 5a is substantially concentric with the first annular weld 5b and is arranged radially outward of the discontinuous welds 5c. The first annular weld 5b is arranged radially inward of the discontinuous welds 5c with respect to the opening 8. The second annular weld 5a overlaps a radial outer edge of the flange 7, and the first annular weld 5b overlaps a radial inner edge of the substrate 2 at the opening 8.

The membrane units as shown in Fig. 2a to 2c can be manufactured by laser welding. In particular, in the embodiment shown in Fig. 2a, the step of laser welding the flange 7 to the substrate 2 at the three discrete welds 5c can be performed before laser welding the first annular weld 5b.

For the embodiments of Fig. 2b and 2c, a further step of laser welding the flange 7 to the substrate 2 at a second annular weld 5a circumferentially surrounding the opening 8 is performed.

In each of these embodiments, the substrate and the membrane 1 having the area 6 are brought into contact such that the area 6 is located in the opening 8. Afterwards, laser welding of the flange 7 to the substrate 2 is performed, wherein the feed rate of the laser along the annular path of the first annular weld is between 200 and 800 mm/s and a laser output is between 300 W and 900 W. Preferably, before the substrate 2 and the flange 7 of the membrane 1 are brought into contact, the surfaces of the substrate 2 and the flange 7 may be roughened by laser structuring.

As shown in Fig. 3a, the membrane 1 may be positioned above the substrate 2, so that the welds 5a, 5b are formed on the upper side of the substrate 2. Hence, the welds are visible in a plan view taken from the above. The piezoelectric element 3 may be provided on the upper side (as indicated in Fig. 3a) or may be provided on the lower side of the substrate 2.

Alternatively, as shown in the embodiment of Fig. 3b, it is conceivable that the membrane 1 is, with its flange 7, positioned beneath the substrate 2. Hence, depending on the positioning of the membrane 1 and the flange 7 of the substrate 2, the welds are formed either on the upper side of the substrate 2 (see Fig. 3a) or on the lower side of the substrate 2 (see Fig. 3b). The piezoelectric element 3 may be provided on the upper side (as indicated in Fig. 3b) or may be provided on the lower side of the substrate 2.

In the embodiments shown in the drawings, the substrate 2 is thicker than the flange 7, in the direction perpendicular to the surface of the substrate 2, i.e. in the direction of the symmetry axis M. Therefore, the welding is performed from the side of the flange 7. In an embodiment which is not shown in the drawings, it is conceivable that the step of welding is performed from a side of the substrate 2, when the substrate 2 is thinner than the flange 7 of the membrane 1 in a direction perpendicular to the surfaces of the substrate 2 and the flange 7 of the membrane 1.

In one embodiment, the membrane 1 is positioned on the opening 8 in the substrate 2. A centring tool or centring aid, for example a stop bar, noses, feed hopper, funnel and so on, and/or alternatively an optical monitoring and positioning system could be used for positioning of the membrane 1 on the substrate 2. After the positioning of membrane 1 and substrate 2, in a further step at least three discontinuous welds 5c arranged in a distance to each other along a circumference of the opening 8, for example, three or more welding points. In the next step, the first annular weld 5b circumferentially surrounding the opening 8 is provided. Optionally, one may create the second annular weld 5a, which is substantially concentric with the first annular weld 5b and is arranged radially outward of it or of the discontinuous welds 5c. In one embodiment, the discontinuous welds 5c may coincide with the second annular weld 5a or the first annular weld 5b. The first annular weld 5b may weld and/or melt the membrane 1 and substrate 2 together. The second annular weld 5a may weld and melt the membrane 1 and substrate 2 together.

In a preferred embodiment, the laser beam is placed on the edge of the opening 8 and is focused on the substrate 2 and/or the membrane 1. The laser beam can be focused on the membrane or the substrate side.

In a preferred embodiment, the laser beam is put on the membrane 1 side, especially when the thickness of the membrane 1 is smaller than the thickness of the substrate 2. In this case, the membrane 1 is melted in its entirety, and only a part of the substrate 2, for example less than half of the thickness of the substrate 2 or even less than one third of the thickness of the substrate 2 is melted. In another preferred embodiment, the laser beam is focused completely on the surrounding substrate 2 and melts the substrate 2 and the membrane 1 together. In this case, the laser beam does not reach only one of the substrate and the membrane, but both of them.

In one embodiment, the laser beam is placed on the flange 7 and is focused on the substrate 2 and/or the membrane 1. The laser beam can be focused on the membrane 1 or the substrate 2 side.

In one embodiment, the laser beam may partly melt the membrane 1 and the substrate 2 so as to form a closed ending of the membrane 1 on the substrate 2. The laser beam may be placed partly on the membrane 1 and partly on the substrate 2. In a preferred embodiment, the laser beam is focused about two thirds on the membrane 1 and about one third on the substrate 2. A good closed ending of the membrane 1 on the substrate 2 with the optional second annular weld 5a can be achieved and extends the life-time of the membrane unit by avoiding any gaps which may be prone to corrosion.

### List of Reference Signs

- 1: membrane
- 2: substrate (support) (optionally including second electrode)
- 3: piezo element
- 4: first electrode
- 5: weld
- 5a: annular weld
- 5b: first annular weld
- 5c: discontinuous welds
- 6: effective area
- 7: flange
- 8: opening
- M: symmetry axis

## Claims

1. A membrane unit for generating an aerosol in an aerosol therapy device, comprising:
a membrane (1) having an area (6) comprising a plurality of through holes for nebulizing a fluid and a flange (7) circumferentially surrounding the area (6); and
a substrate (2) having an opening (8), wherein the flange (7) of the membrane is welded to the substrate (2) so that the area (6) is located in the opening (8), wherein the weld (5) comprises at least three discontinuous welds (5c) arranged in a distance to each other along a circumference of the opening (8) and a first annular weld (5b) circumferentially surrounding the opening (8).

2. The membrane unit according to claim 1, wherein the discontinuous welds (5c) are arranged on a common circle concentric with the first annular weld (5b).

3. The membrane unit according to claim 1 or 2, wherein the first annular weld (5b) is arranged radially inward of the discontinuous welds (5c) with respect to the opening (8).

4. The membrane unit according to any one of the preceding claims, further comprising a second annular weld (5a) substantially concentric with the first annular weld (5b).

5. The membrane unit according to claim 4, wherein the second annular weld (5a) is arranged radially outward of the discontinuous welds (5c).

6. A membrane unit for generating an aerosol in an aerosol therapy device, comprising:
a membrane (1) having an area (6) comprising a plurality of through holes for nebulizing a fluid and a flange (7) circumferentially surrounding the area (6); and
a substrate (2) having an opening (8), wherein the flange (7) of the membrane is welded to the substrate (2) so that the area (6) is located in the opening (8), wherein the weld (5) comprises a first annular weld (5b) circumferentially surrounding the opening (8) and a second annular weld (5a) substantially concentric with the first annular weld (5b).

7. The membrane unit according to any of the preceding claims, wherein the second annular weld (5a) overlaps a radial outer edge of the flange (7) in a plan view and/or the first annular weld (5b) overlaps a radial inner edge of the substrate (2) at the opening (8) in a plan view.

8. The membrane unit according to any one of the preceding claims, wherein the welds (5) are laser welds.

9. An aerosol therapy device for generating an aerosol, comprising the membrane unit according to any of the preceding claims.

10. Method of manufacturing a membrane unit of an aerosol generator, the method comprising the steps of:
bringing a substrate (2) having an opening (8), and a membrane (1) having an area (6) comprising a plurality of through holes for nebulizing a fluid and a flange (7) circumferentially surrounding the area in surface contact so that the area (6) is located in the opening (8),
laser welding the flange (7) to the substrate (2) at a first annular weld (5b) circumferentially surrounding the opening (8), wherein preferably a feed rate of the laser along the annular path of the first annular weld (5a, 5b) is between 200 and 800 mm/s and a laser output is between 300W and 900W.

11. Method according to claim 10, further comprising the step of laser welding the flange (7) to the substrate (2) at at least three discontinuous welds (5c) arranged around the opening (8) in a circumferential direction before laser welding the first annular weld (5b).

12. Method according to claim 10 or 11, further comprising the step of laser welding the flange (7) to the substrate (2) at a second annular weld (5a) circumferentially surrounding the opening (8).

13. Method according to any one of claims 10 to 12, wherein the laser used for laser welding is a multimode laser having a scanner optic.

14. Method according to any one of claims 10 to 13, wherein at least one of the surfaces of the substrate (2) and the flange (7) of the membrane to be brought in contact is roughened by laser structuring.

15. Method according to any one of claims 10 to 14, wherein the substrate (2) is thicker than the flange (7) of the membrane (1) in a direction perpendicular to the surfaces of the substrate (2) and the flange (7) of the membrane to be brought in surface contact and the step of laser welding is performed from a side of the flange (7) of the membrane.
